# EUROPEAN PATENT APPLICATION

(11) **EP 1 163 877 A1**
(43) Date of publication of application: **19.12.2001**
(21) Application number: 00112680.4
(22) Date of filing: 15.06.2000
(51) Int. Cl.: A61B 5/113, A61B 5/00

(54) **System for body activity detection and processing**

(71) Applicant: THE PROCTER & GAMBLE COMPANY, Cincinnati, Ohio 45202 (US)
(72) Inventor: Barron, Bradford S., 1780 Wemmel (BE); Boswell, Emily Charlotte, Isleworth Middlesex TW7 7PP (GB); Dauger-Strauss, Corrine, 1040 Brussels (BE); Deflander, Joseph Fernand, 2990 Wespelaar (BE); MacGilp, Neil Archibald, Bankfield Surrey, GU5 0BX (GB); Van den Wouwen, Chris, 2100 Deurne (BE)
(74) Representative: Hirsch, Uwe Thomas

(57) **Abstract**

A system for monitoring body activity having a device arranged for stand-alone attachment to a body in use. The device comprises: an actimetry sensor (11) for measuring body activity, and storage means (12) for receiving data from the actimetry sensor and storing it. Means analyses the stored data to provide advisory information and a display (4) displays the advisory information to a user.

## Description

### Field of the invention

This invention relates to the detection of body activity, such as sleep patterns, and the analysis of data related to such functions for provision to a user.

### Background

In recent years there has been much study of body functions, such as sleep activity, and associated analysis of the relevance of such functions to the general health of the body and the body's need for appropriate body functions (such as sleep patterns) to occur on a regular basis for adequate periods of time. As part of this research numerous devices have been proposed to assist in such measurement and analysis.

For example, WO-A-9714354 discloses a device and corresponding method which collects data for analysing sleep disturbances so that such data can be interpreted by a specialist at a future date.

However, this type of device requires operation by a highly skilled user and provides analysis which is difficult to interpret by anybody other than a specialist, as well as being expensive and sometimes unreliable. Furthermore, it is unable to provide a detailed history over an extended time period for an individual.

Other systems are uncomfortable, cannot be worn for extended periods and/or cannot be worn without restricting body movement.

### Summery of the invention

According to the present invention there is provided a system for monitoring body activity comprising:
a monitoring device arranged for stand-alone attachment to a body in use, the device comprising: an actimetry sensor for measuring body activity; storage means for receiving data from the actimetry sensor and storing it; and data transmitting means for transmitting data from the storage means;
data receiving means separate from the device for receiving the data;
means for analysing the received data to provide advisory information; and
means for displaying the advisory information to a user.

The actimetry sensor may be an accelerometer such as a piezoelectric accelerometer, or may be a simple motion sensor or a tilt switch, for example.

The body activity being monitored may be sleep.

The storage means may store data from the actimetry sensor together with temporal information. In such a case, the means for analysing the stored data provides processing based upon both body activity information and temporal information to provide advisory information to the user.

The advisory information provided to the user may include an indication of the quality of the activity, such as the quantity or quality of the sleep, whether or not the duration of the activity is sufficient, an indication as to whether the total amount of the activity over an extended period is acceptable, as well other data related to other long term body activity, for example.

The device can be configured to detect activity during the day. Daytime activity may be then analysed to indicate the most active and least active time during the day, so that information can be gathered in terms of the best time of day to attend a meeting, carry out exercise etc. The body activity that is measured can, as well as being actual time slept, be the number of awakenings, an indication as to how the intermittent sleep was, time taken before sleep, the number of and length of sleep interruption, sleep proficiency, the number minutes immobile/moving, etc. A selection or all of this information can be provided to identify the least and most active times during the day.

The system may include an input on the device or analysing means for receiving input data from a user, such as desired time to go to sleep, the need to awake early for a particular event, as well as possible information relating to the age of the user, their sex, as well as optionally additional information such as what they perceive their energy level to be.

There may be provided a further sensor or sensors on the device to measure body pulse rate variability, blood pressure or other body activities such as respiration, eyelid movement. In this case, sleep phases, such as REM, slow light sleep, slow deep sleep, or paradoxical sleep maybe monitored.

The device may be configured in the style of a wrist watch, and may be arranged with a display to receive data from the analysing means to provide the advisory information as well as to provide additional information to a user, such as time and date information.

The means for displaying the advisory information may be a liquid crystal display, plasma display, etc.

The data transmitting means may be an infra red or other form of wireless transmitter. Alternatively, it may be a wire data connection for insertion into a receiving terminal acting as the data receiving means. In this latter case the system would be arranged for a user to remove the monitoring device and place it in the data receiving means in order to activate the transmission of data to the receiving means and onto the analysing means.

The analysing means may be an appropriately configured PC or may be a dedicated processing device. The display means may be formed as part of the device, in which case the device also has second data receiving means for receiving data from the analysing means. Alternatively, the display may be formed as part of the analysing means and separate from the device.

The analysing means may have further means for connecting it to a terminal remote from the system, so that the analysing means may communicate with an external reference source, which may be a larger database of information or may be a specialised human reference. In this case, the connection to the remote source of information may be via an internet-type connection.

### Brief description of the figures

One example of the present invention will now be described with reference to the accompanying drawings, in which:
Figure 1 is a schematic perspective view of components of a system according to the present invention;
Figure 2 is a schematic diagram showing some of the functionality of a system according to the invention;
Figure 3 is a diagram showing two possible displays from the system; and
Figure 4 is a schematic diagram of the internal components of the system of Figure 1.

### Detailed description of an example

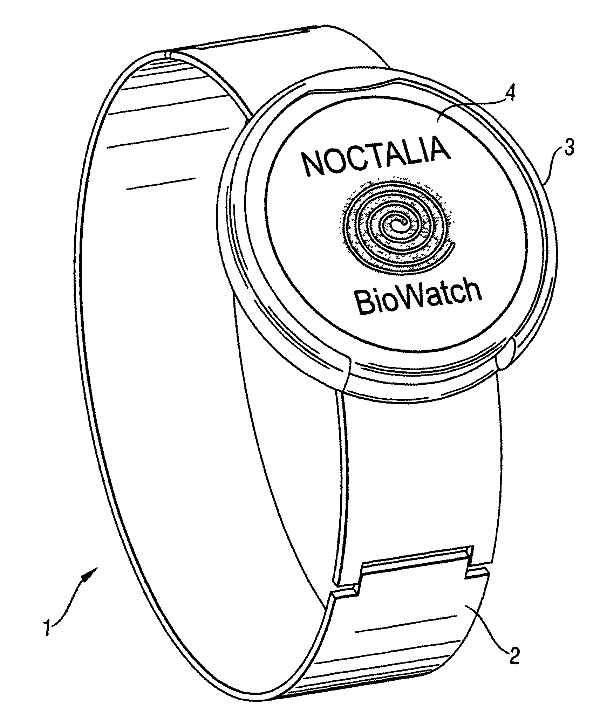

Referring to Figure 1, a system according to the present invention has, in this example, a monitoring device configured as a wrist watch-style device, with a strap 2 and a component-containing housing 3. On the outer surface of the housing 3 is an optional display 4, which in this case is a liquid crystal display.

The system further comprises a receiving device, often referred to as a docking port, which in use can receive the monitoring device 1 and connect with it to retrieve data from the device 1. The receiving device 5 is, in turn, connected to an analysing device 6, which in this case is an appropriately configured PC, although it could be a dedicated piece of hardware. In this case the receiving device 5 and analysing device 6 are shown as separate components, although this may not necessarily be the case.

The analysing device 6 has its own display 7, and may optionally have the ability to connect to a remote terminal (not shown) via an Internet link or some other form of communication device.

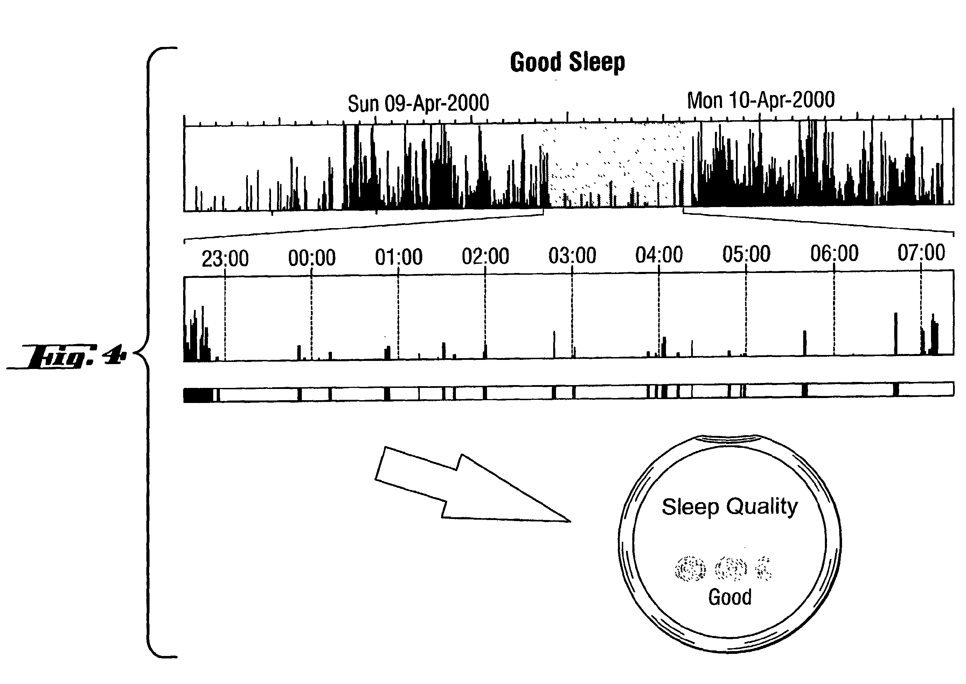

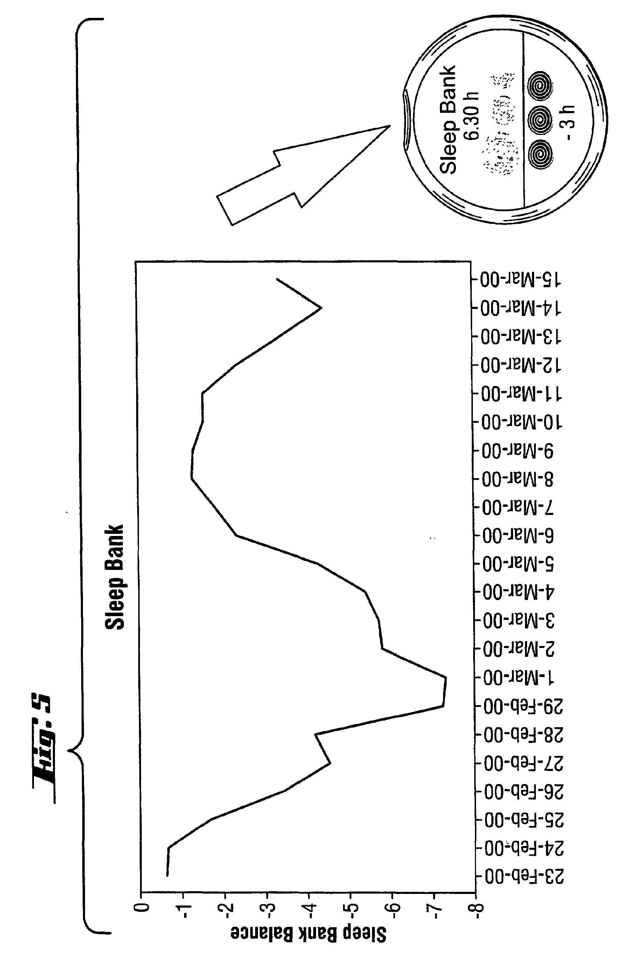

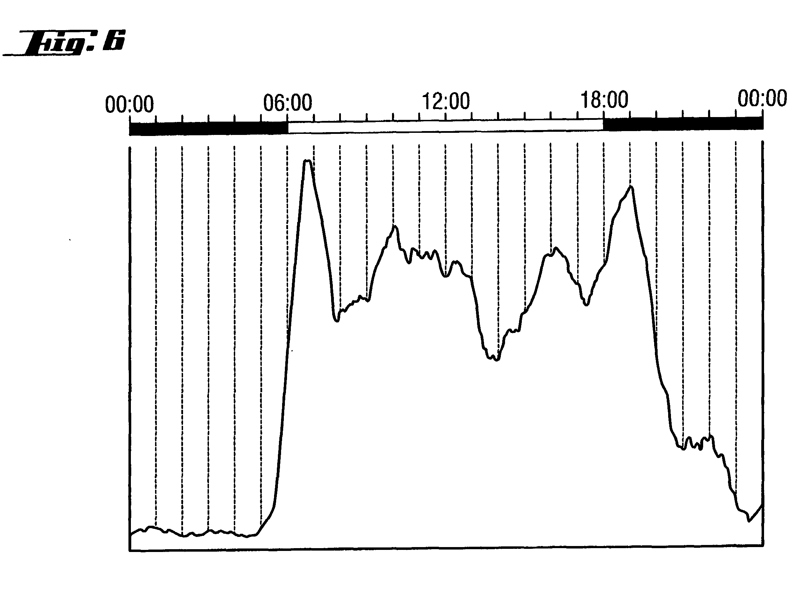

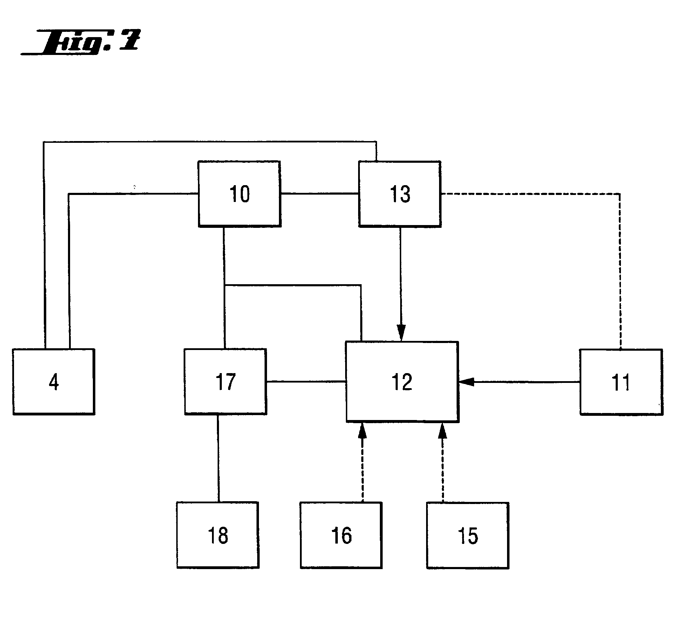

Referring to Figure 4, the device 1 of Figure 1 has a number of internal components. The device 1 is powered by a battery 10 (or another power supply) which supplies power to the other components of the device 1. An actimetry sensor 11 detects motion in the device 1 and hence motion in the body to which the device 1 is attached in use. The data from the actimetry sensor 11 is passed to a memory 12. A clock 13 also provides temporal data to the memory 12 and to the actimetry sensor 11 if necessary, as well as optionally to the display 4. In addition to the actimetry sensor 11 the device 1 may further comprise additional sensors 15, 16, which may detect blood pressure, pulse rate variation etc.

Data from these additional optional sensors 15, 16 may also be forwarded to the memory 12.

As described above, the monitoring device 1 has the ability to send data to the receiving means 5 via a transmitter 20, in this case through a fixed connection between the two when the user places the monitoring device 1 in the receiving device 5. As an alternative, the monitoring device 4 may transmit and/or receive data from the receiving means 5 via a wireless link such as an infra red link. In this latter case data from the memory 12 can be requested from analysing means 6, either on a regulated intermittent, continuous, or on a user-requested basis.

A wide variety of different forms of analysis may be performed by the analysing means 6.

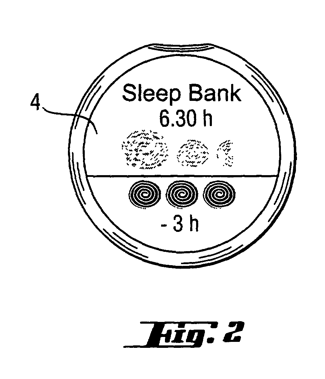

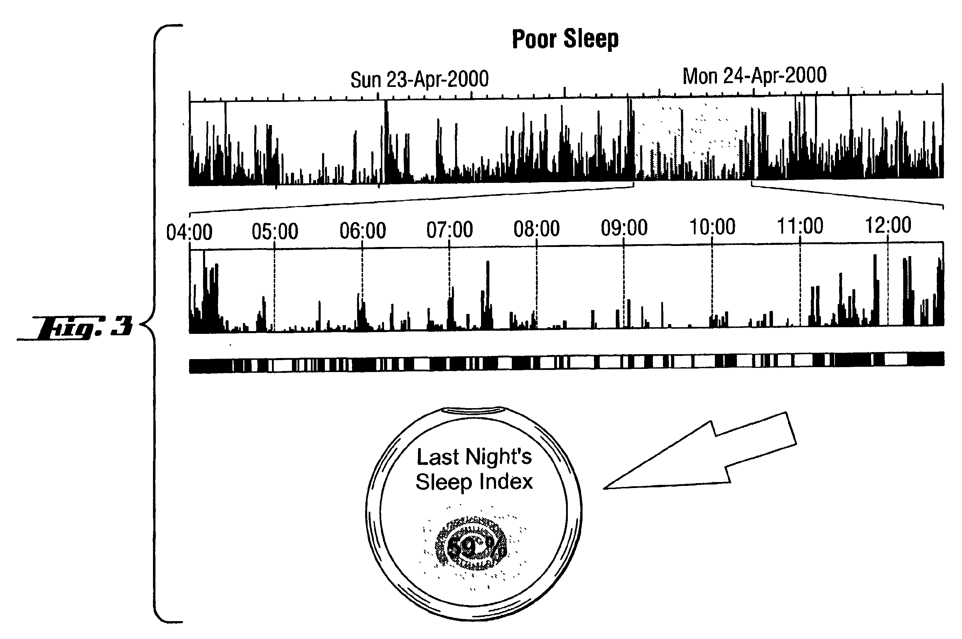

Examples of the types of analysis that may be performed will now be described with reference to Figures 2 and 3.

The actimetry sensor 11 may provide information in relation to sleep duration and the type of sleep to the analysing means 6. This information can be analysed by the analysing means 6 to provide information to the display 4 in terms simply of the total number of hours of sleep obtained, although it may provide additional information in relation to the quality of the sleep and the expected value of that sleep in terms of an "energy bank". By using data stored in the memory 12 or as the base unit in an additional memory (not shown) over a number of days, weeks or months, the analysing means 6 may also provide information indicative of accumulated sleep deficit or sleep excess. As mentioned above, the data can be provided to a user as and when requested, and is arranged to be provided in a very simple format so that it does not need complex interpretation.

The analysing means 6 may employ, for example, the Stanford sleep scale in order to score the monitored, sleep and provide relevant information to the user and to some subject we input from the user. The scale defines different levels of sleepiness as follows;
1 - feeling active, vital, alert, wide awake.
2 - functioning at a high level, not at peak.
3 - relaxed, not full alertness, responsive.
4 - a little soggy, not at peak, let down.
5 - tired, losing interest, slowed down.
6 - drowsy, prefer to be lying down.
7 - almost in a reverie, hard to stay awake.

This scale can be shown to a user so that the user can input an indication of how tired they consider themselves to be.

For example, the user could be prompted to input an indication as to how they feel when they wake up, with an indication as to the reasons for their feelings being provided by the analysing means 17 from the data collected.

In another example, such an input could be employed during the initial weeks of employing the device to help the system determined whether or not the user is sleeping for the right amount of time to them. For example, on the first day of wearing the device the system may prompt the user to indicate how much sleep they consider they need. It could then provide information as to the average sleep requirement for someone of their age and sex. However, as the requirements vary from user to user, the system can then monitor sleep over a given period and prompt the user for feedback, not only at the time during the day in order to form a sleep diary in the memory of the system. The system may then be configured to adapt the indications that it gives the user based upon the feedback and wake the user at the appropriate time, and then employing a sleep bank once the user's particular requirements have been determined.

The device 1 may have an alarm 18, which can be used simply to wake the user, in the manner of a normal wrist watch alarm, although it may be activated by the analysing means 6, when it is detected that an appropriate type of sleep is occurring to ensure gentle waking of the user.

If additional sensors 15, 16 are provided then additional analysis can be performed dependent upon the type of sensor to provide additional or more detailed and accurate information to the user. If the sensors detect parameters external to the body, such as light, location, sound, air temperature, humidity, barometric pressure, then this information may be compared with information relating to body activity in order to adjust their information. If the sensors determine additional body activity, and detect one or more muscle tonus, skin temperature, galvanic skin response, etc then additional analysis of the quality of the sleep may be provided.

As a further example, if a blood pressure sensor is employed then additional indications related to general levels of health and activity not specifically related to sleep alone can be provided by the analysing means. If a pulse rate variability detector is employed then this can assist in determining the type of sleep detected, and can provide further information in relation to whether an acceptable level of aerobic exercise has been performed within the allotted time period, whether it be a day, a week or a month.

If the device 1 provides some form of "sleep bank" indication over a period of time, then the sleep bank may calculate the information to be provided to the user by including a formula such as:
sleep bank (i) = sleep bank (i-1) + (sleep (i) - need) where sleep bank is accumulative of sleep balance on day i, sleep is sleep achieved on the night before day i and need is sleep need (which can change dependent upon other measured parameters, or upon stored data, or can be set manually).

In the case when the analysing means 6 has a line to a remote station, more complex analysis can be performed and it may be possible for the analysing means 6 also to request and obtain data from a human specialist or an extended database or an extended database so that additional information can be provided to the user.

In addition, the system enables the storage of long term data in such a manner that it can be tracked to give a high quality user history for treatment, as well as for identifying long term trends that would not come to light in a short term analysis.

## Claims

1. A system for monitoring body activity comprising:
a monitoring device arranged for stand-alone attachment to a body in use, the device comprising: an actimetry sensor for measuring body activity; storage means for receiving data from the actimetry sensor and storing it; and data transmitting means for transmitting data from the storage means;
data receiving means separate from the device for receiving the data;
means for analysing the received data to provide advisory information; and
means for displaying the advisory information to a user.

2. The system of claim 1, wherein the actimetry sensor is an accelerometer such as a piezoelectric accelerometer.

3. The system of claim 1 or 2, wherein the body activity being monitored is sleep.

4. The system of any preceding claim, wherein the storage means stores data from the actimetry sensor together with temporal information.

5. The system of claim 4, wherein the means for analysing the stored data provides processing based upon both body activity information and temporal information.

6. The system of any preceding claim, wherein the advisory information provided to the user includes an indication of the quality of the activity, including at least one of the quality of the sleep, whether or not the duration of the activity is sufficient, an indication as to whether the totalled amount of the activity over an extended period is acceptable, or other data related to other long term body activity.

7. The system of any preceding claim, wherein the actimetry sensor also measures body pulse rate, and/or blood pressure.

8. The system of claim 7 wherein, sleep phases of REM, slow light sleep, slow deep sleep, or paradoxical sleep are monitored.

9. The system of any preceding claim, wherein the device may be configured in the style of a wrist watch.

10. The system of claim 9, wherein the device includes an alarm.

11. The system of any preceding claim, wherein the means for displaying the advisory information is a liquid crystal display.

12. The system of claim 9, 10 or 11, wherein the display is attached to the monitoring device.

13. The system according to any preceding claim, wherein the analysing means has a communication link with a remote source of data.

14. The system of claim 13, wherein the remote station enables the input of additional data from a human operator.

15. The system of any preceding claim, wherein the receiving means and the transmitting means form a wireless communication link.

16. A system according to any one of claims 1 to 14, wherein the data receiving means is part of a docking station for receiving the device.

17. A system according to any preceding claim, wherein the analysing means performs analysis on the basis of data from a source other than the body.

18. A system according to claim 17, wherein the source is a further sensor for detecting data relating to the environment in which the body is placed.
